# EUROPEAN PATENT SPECIFICATION

(11) **EP 0 325 191 B1**
(45) Date of publication and mention of the grant of the patent: **13.09.1995**
(21) Application number: 89100675.1
(22) Date of filing: 17.01.1989
(51) Int. Cl.: A61K 38/46, A61K 39/395, A61K 39/02, C12N 9/22

(54) **Composition and method for protecting against diseases caused by microorganisms**
Zusammensetzung und Verfahren zum Schutz gegen durch Mikroorganismen verursachte Krankheiten
Composition et procédé pour protéger contre des maladies causées par des micro-organismes

(30) Priority: 20.01.1988 US 146256
(43) Date of publication of application: 26.07.1989
(73) Proprietor: ML TECHNOLOGY VENTURES, L.P., New York New York 10080 (US)
(72) Inventor: Faulds, Daryl, Millbrae, CA (US); Vishoot, Mimi, Millbrae, CA (US)
(74) Representative: Perry, Robert Edward

(56) References cited:
- WO-A-86/00019

## Description

This invention relates to a composition and method for protecting against diseases caused by microorganisms. Still more particularly, the invention is directed to a composition and method for protecting against diseases caused by microorganisms which lack biosynthetic pathways for nucleic acid precursors in particular mycoplasma organisms and most particularly mycoplasmal pneumonia of swine caused by M. hyopneumoniae.

There are diseases which are caused by various microorganisms; for example mycoplasma organisms. The disease caused by Mycoplasma hyopneumoniae (in particular in swine), occurs throughout the world and is a disease associated with the loss of swine. The disease generally results in inefficient, stunted and sickly animals, and effected swine and often prone to secondary infection by opportunistic microorganisms.

There have been numerous attempts to provide a vaccine for protecting swine against mycoplasmal pneumonia; however, such vaccines have not been successful.

Kristensen et al, Am. J. Vet. Res. 42:784 (1981), found no protection of swine against mycoplasmal pneumonia after injection with heat-inactivated cells of Mycoplasma hyopneumoniae.

Etheride et al, Res. Vet. Sci. 33:188 (1982), found incomplete protection against lung colonisation by Mycoplasma when a live vaccine was given intravenously, subcutaneously or intraperitoneally.

Ross et al, Am. J. Vet. Res. 45:1899 (1984), disclosed that the use of Mycoplasma hyopneumoniae extracts prepared by a freeze-thaw procedure provided only variable protection and, in some instances, enhanced lesion development. Ross et al also claimed that injection of such agent into swine gave some level of protection against an intratracheal challenge exposure consisting of 4 ml of supernate from a 10% suspension of pneumonic lung containing strain VPP-11 combined with 1 ml of a 24-hour culture of 15 to 20 passages of the same strain.

Bartholeyns et al, Europ. J. Cancer 15(4):85-91 (1979), discloses that a dimer of bovine pancreatic RNase A blocks the proliferation of tumoural cells.

According to the present invention, a composition for protecting an animal against a disease caused by a microorganism which lacks biosynthetic pathways for nucleic acid precursors and which is a mycoplasma, acholeplasma, spiroplasma, ureaplasma or anaeroplasma, comprises:
(a) a member selected from (i) nucleases which produce antibodies having paratopes which recognise an extrinsic nuclease of the microorganism, and (ii) antibodies having paratopes which recognise an extrinsic nuclease of the microorganism; and
(b) a physiologically-acceptable carrier.

According to a further aspect of the invention, the said nucleases/antibodies are used for the preparation of a medicament for protecting an animal against such a disease.

The extrinsic nuclease is any nuclease which is secreted by and/or exposed on an external surface of the microorganism against which protection is to be afforded. The nuclease may be a deoxyribonuclease (DNA-degrading enzyme or DNase) and/or a ribonuclease (RNA-degrading enzyme or RNase) which produces antibodies which recognise, respectively, extrinsic RNase or DNase. The RNase or DNase which is employed may or may not have enzymatic activity as long as it is capable of producing antibodies of the type described.

Although the present invention is not to be limited by any explanation, it is believed that certain organisms which are not capable of synthesising nucleic acid precursors require the presence of nuclease to obtain purines and pyrimidines required for growth and infection; and that nuclease secreted by and/or exposed on the surface of the cell functions to produce purines and pyrimidines from nucleic acids provided by the host cells. The administering of an appropriate nuclease which specifically binds or remove the extrinsic nuclease thereby provides protection against growth of and infection by the microorganism. Thus, by inducing in an animal the production of antibodies which recognise extrinsic RNase and/or extrinsic DNase of the microorganism against which protection is to be afforded, growth of and infection by such microorganism may be eliminated or retarded.

In some cases, protection may be afforded by using only RNase or only DNase. In other cases, it may be necessary to administer both RNase and DNase. As should be apparent, such use is dependent on whether or not the microorganism requires RNase, DNase or both to produce the purines and pyrimidines required for growth and infection.

The nuclease used for providing protection may be obtained from the microorganism against which protection is sought by recovering extrinsic nuclease from the organism. As a further alternative, a recombinant DNA molecule encoding an amino-acid sequence having one or more antigenic determinants (epitopes) of the extrinsic nuclease may be employed for producing the nuclease, by genetic engineering techniques. The nuclease produced by genetic engineering techniques should produce antibodies which recognise the extrinsic nuclease, i.e. an immune response.

In accordance with a preferred embodiment, the present invention is directed to protecting against diseases caused by mycoplasma organisms. The nuclease is preferably a DNase, and the DNase is preferably a DNA endonuclease.

For use in the invention, a vaccine may be provided for protecting animals, in particular non-human animals, against diseases caused by microorganisms of the type described; in particular, in order to protect swine against mycoplasma organism, a nuclease is used which has one or more epitopes of the extrinsic DNase present in porcine mycoplasma. More particularly, for protecting swine against mycoplasmal pneumonia, a vaccine comprises a nuclease, such as DNase, in combination with a suitable physiologically acceptable vehicle; e.g. a carrier and/or adjuvant.

The present invention will be further described, for the purposes of illustration only, with respect to protecting against diseases caused by mycoplasma, by the use of DNase in a vaccine. The DNase may be derived from the Mycoplasma organism; i.e. the DNase may be recovered from the membrane of the Mycoplasma organism by use of a mild detergent, such as a non-ionic detergent, to solubilise the DNase.

The non-ionic detergent is employed in amounts which are sufficient to solubilise the DNase. In general, the weight ratio of non-ionic detergent to Mycoplasma organism portion which is subjected to treatment is from 0.05:1 to 10.0:1, and preferably from about 0.5 to 1 to 5.0 to 1. The treatment is generally effected at a temperature which does not exceed 40°C with the temperature most generally being in the order of from 1°C to 37°C.

The treatment is for a time sufficient to effect solubilization of the DNase and in general, such time is in the order of from 0.5 to 18 hours; however, in some cases, longer or shorter times may be employed.

The solution employed to solubilize the DNase generally has an ionic strength of from 0.05 to 1.0 M salt. A preferred solubilizing agent contains 0.2M sodium ion.

As hereinabove indicated, in general, the membrane of the Mycoplasma organism is subject to such treatment. Such membrane may be obtained by disrupting the organism by procedures generally known in the art, such as a freeze-thaw cycle; sonication; etc. Alternatively, the DNase may be derived from the whole organism. The selection of a suitable procedure is deemed to be within the scope of those skilled in the art from the teachings herein.

DNase may also be recovered from the supernatant of a mycoplasma cell culture; e.g., by precipitating DNase from the cell supernatant with ammonium sulphate.

As a further alternative, the DNase or fragment or derivative thereof may be obtained by genetic engineering techniques; for example by use of a recombinant DNA molecule which encodes for a nuclease or fragment or derivative thereof (in particular a DNase or fragment or derivative thereof) which produces antibodies having paratopes which recognize an extrinsic nuclease of the microorganism against which protection is sought. Such nuclease is employed in the vaccine in an amount effective to provide protection against a disease caused by the microorganisms, e.g., a Mycoplasma organism and in particular mycoplasmal pneumonia caused by M. hyopneumoniae.

In general, the vaccine contains at least 5 micrograms per dose and preferably at least 100 micrograms per dose of such nuclease, such as DNase and/or fragment or derivative. In most cases, the vaccine does not include the nuclease, and/or fragment or derivative in an amount greater than 20 milligrams.

If multiple doses are employed, in general, the vaccine would not be administered in an amount which exceeds 3 doses over 8 weeks.

The term "protection" or "protecting" when used with respect to the vaccine or methods described herein means that the vaccine prevents a disease caused by the organism, in particular mycoplasmal pneumonia caused by M. hyopneumoniae and/or reduces the severity of a disease caused by the organism, in particular mycoplasmal pneumonia caused by M. hyopneumoniae.

The vehicle which is employed in conjunction with the nuclease may by any one of a wide variety of vehicles. As representative examples of suitable vehicles, there may be mentioned: mineral oil, alum, synthetic polymers, etc. Vehicles for vaccines are well known in the art and the selection of a suitable vehicle is deemed to be within the scope of those skilled in the art from the teachings herein. The selection of a suitable vehicle is also dependent upon the manner in which the vaccine is to be administered. The vaccine may be in the form of an injectable dose and may be administered intra-muscularly, intravenously intratracheally, intraperitoneally, intranasally, e,g. as an inhalent, or intraoccularly, or by sub-cutaneous administration. It is also possible to administer the vaccine orally by mixing the active components with feed or water; providing a tablet form, etc.

Other means for administering the vaccine should be apparent to those skilled in the art from the teachings herein; accordingly, the scope of the invention is not limited to a particular delivery form.

It is also to be understood that the vaccine may include active components or adjuvants in addition to the nuclease or fragments or derivatives hereinabove described.

It is also to be understood that the vaccine may include a combination of active components; e.g., a mixture of appropriate nuclease fragments or a mixture of nuclease and nuclease fragment, etc.

The vaccine is generally employed in non-human animals which are susceptible to diseases caused by Mycoplasma organisms, particularly mycoplasmal pneumonia caused by M. hyopneumoniae, and in particular in swine, and bovines.

The antibody may be produced from a nuclease (RNase or DNase) having the characteristics hereinabove described by procedures known in the art and such antibody may be either a monoclonal or polyclonal antibody. The antibody or appropriate fraction or derivative thereof may be employed for affording protection.

The antibody may be employed in a composition and administered as hereinabove described with reference to the use of a nuclease. In using an antibody, the vaccine generally contains at least 5 micrograms per dose and preferably at least 50 micrograms per dose of such antibody. In most cases, the unit dosage dose does not exceed 20 milligrams.

Thus, in accordance with this aspect of the present invention, antibodies which recognize extrinsic nuclease of the microorganism against which the animal is to be protected are produced externally of the animal and such antibodies or fragments or derivative thereof, are administered to the animal to afford protection to the animal.

DNase for use in the invention may be recovered directly from the M. hyopneumoniae organism, as hereinabove described, and purified to obtain substantially pure DNase.

It is also possible to produce such DNase by genetic engineering principles, in which case the M. hyopneumoniae DNase could be produced in an organism other than M. hyopneumoniae, e.g., by use of a recombinant DNA molecule encoding an amino acid sequence which produces antibodies which recognize extrinsic DNase of M. hyopneumoniae.

The extrinsic M. hyopneumoniae DNase is further characterized by:
A. Thermal lability. The cell surface DNase is inactivated by incubation at 56°C for 10 minutes.
B. Trypsin sensitivity. The cell surface DNase activity is abolished by treatment of intact cells with trypsin.
C. Apparent molecular weight. When subjected to SDS polyacrylamide gel electrophoresis the enzyme runs as a doublet with an apparent molecular weight of 35 to 40 kDa.
D. Ion exchange characteristics. The DNase binds to DEAE-cellulose in 0.0175 M NaCl and may be eluted with a NaCl gradient of 0.0175 M to 0.5 M. The DNase binds to phosphocellulose in 0.0175 M NaCl and may be eluted with a NaCl gradient of 0.0175 M to 1.0 M.

A composition of the invention may comprise, in addition to the nuclease, one or more proteins which are capable of eliciting an antibody which recognises a M. hyopneumoniae antigen which lacks immunosuppressive activity. A vaccine of this type, essentially free of M. hyopneumoniae antigens which have immunosuppressive activity, may be used for protection against mycoplasmal pneumonia. The weight ratio of protein to nuclease may be from 100:1 to 1:40, and preferably from 4:1 to 1:4.

A vaccine of the invention may also comprise the one or more proteins and antibodies for use in the invention. The weight ratio of antibody to protein may be from 1000:1 to 10,000:1.

It has been found that Mycoplasma hyopneumoniae organisms include a plurality of antigens, some of which have immunosuppressive activity and some of which lack immunosuppressive activity. It has also been found that the Mycoplasma hyopneumoniae antigens which lack immunosuppressive activity are suitable for use in a vaccine effective against mycoplasmal pneumonia.

The proteins which elicit an antibody which recognises a Mycoplasma hyopneumoniae antigen may be of recombinant or natural origin. If of natural origin, they may be obtained. from Mycoplasma hyopneumoniae organisms, with selective recovery of the Mycoplasma hyopneumoniae antigens which lack immunosuppressive activity.

For example, a procedure for determining whether or not a compound or composition possesses immunosuppressive activity is reported by Suter et al, Infect. and Immun. 49:615 (1985).

In accordance with one method, the membrane of the Mycoplasma hyopneumoniae organism may be treated with a mild detergent, and in particular a non-ionic detergent to produce both a soluble and insoluble fraction. Applicant has found that the insoluble fraction includes Mycoplasma hyopneumoniae antigens which lack immunosuppressive activity, whereas the soluble fraction, obtained by treatment with a mild detergent, includes Mycoplasma hyopneumoniae antigens which lack immunosuppressive activity, as well as materials which have immunosuppressive activity. The insoluble fraction obtained by treating the membrane from Mycoplasma hyopneumoniae organism with a mild detergent may be employed in conjunction with at least one nuclease of Mycoplasma hyopneumoniae, for formulating a vaccine for protecting animals against mycoplasmal pneumonia or, alternatively, the soluble fraction may be treated to remove materials which have immunosuppressive activity, whereby such fraction may be employed in conjunction with at least one nuclease of M. hyo for formulating a vaccine.

As a further alternative, the membrane derived from Mycoplasma hyopneumoniae organism may be treated with other agents to selectively solubilize the surface antigens which possess immunosuppressive activity and thereby provide an insoluble fraction containing Mycoplasma hyopneumoniae antigen(s) which lack immunosuppressive activity. As representative examples of other solubilizing agents, these may be mentioned: n-octylglucoside; sodium deoxycholate; CHAPS, i.e. 3-[(3-Cholamidopropyl) dimethylammonio]-1-propanesulfonate.

As another example of a procedure for obtaining Mycoplasma hyopneumoniae antigens which lack immunosuppressive activity, there may be mentioned electrophoretic procedures for separating the various Mycoplasma hyopneumoniae membrane antigens so as to obtain one or more Mycoplasma hyopneumoniae antigens which lack immunosuppressive activity.

The above procedures and others should be apparent to those skilled in the art from the teachings herein.

The Mycoplasma hyopneumoniae antigens which lack immunosuppressive activity and which are employed in conjunction with at least one nuclease or fragment and/or derivative thereof for formulating a vaccine for protecting against mycoplasmal pneumonia generally have a molecular weight of at least 10kDa and generally no greater than 350kDa. In most cases, such antigens have a molecular weight of from 22kDa to 121kDa. The vaccine may include one or more of such antigens or fragments thereof. As should be apparent, a fragment would have a molecular weight lower than the antigen from which it is derived. In an embodiment, the vaccine contains one or more of the antigens having the following molecular weights: 22.5 kDa; 34 kDa; 36 kDa; 41 kDa; 44 kDa; 48 kDa; 52 kDa; 64 kDa; 74.5kDa; 79kDa; 88.5kDa; 96.5kDa and 121kDa.

The molecular weights for characterizing the antigen(s) may be obtained by discontinuous polyacrylamide gel electrophoresis using the SDS buffer system described by Laemmli, Nature 227; 680-85 (London 1970) with an acrylamide concentration of 12% and a bis-acrylamide to acrylamide ratio of 0.8:30.

The non-ionic detergent is employed in amounts which are sufficient to solubilize the Mycoplasma hyopneumoniae antigens which possess immunosuppressive activity. In general, the weight ratio of non-ionic detergent to Mycoplasma hyopneumoniae organism portion which is subjected to treatment is from 10 to 1 to about 0.05 to 1, and preferably from about 5.0 to 1 to 0.5 to 1. The treatment is generally effected at a temperature which does not exceed 40°C, with the temperature most generally being in the order of from 0°C to 37°C.

The treatment is for a time sufficient to effect solubilization of the Mycoplasma hyopneumoniae antigens which possess immunosuppressive activity, and in general, such time as in the order of from 0.5 to 12 hours, however, in some cases, longer or shorter times may be employed.

The solution employed to solubilize the antigen(s) generally has an ionic strength of from 0.05 to 1.0 M. A preferred solubilizing agent contains 0.2M sodium ion.

As hereinabove indicated, in general, the membrane of the Mycoplasma hyopneumoniae organism is subject to such treatment. Such membrane may be obtained by disrupting the organism by procedures generally known in the art, such as a freeze-thaw cycle; sonication; etc. Alternatively, the antigen(s) may be derived from the whole organism. The selection of a suitable procedure is deemed to be within the scope of those skilled in the art from the teachings herein.

In addition to obtaining Mycoplasma hyopneumoniae antigens and/or fragments thereof by treating Mycoplasma hyopneumoniae organisms, it is to be understood that the antigens and/or fragments thereof may be recombinant in origin and obtained by various methods of genetic engineering.

A vaccine for protection against mycoplasmal pneumonia, and in particular for protecting swine against mycoplasmal pneumonia, is comprised of one or more of the M. hyopneumoniae antigens which lack immunosuppressive activity or fragments or derivatives thereof, and at least one nuclease (or fragment or derivative thereof) of Mycoplasma hyopneumoniae as hereinabove described, in combination with a suitable physiologically acceptable vehicle such as a carrier or adjuvant. The Mycoplasma hyopneumoniae antigen(s) and/or fragment(s) thereof and said at least one nuclease and fragment(s) and/or derivative(s) thereof are employed in the vaccine in amounts effective to provide protection against mycoplasmal pneumonia.

The vaccine may contain the Mycoplasma hyopneumoniae antigen and nuclease in a ratio of antigen to nuclease of from about 100:1 to about 1:40, preferably of from about 4:1 to about 1:4. Each dose of the vaccine may contain from about 10µg to about 200µg of M. hyo. antigen and from about 0.5µg to about 1,000 µg of DNase, preferably from about 25 µg to about 100µg of antigen and from about 10µg to about 200µg of DNase. If multiple doses are given, in general the number of doses would not exceed three doses over an eight week period. The present invention will be further described with respect to the following examples; however, the scope of the invention is not to be limited thereby:

### EXAMPLE 1

Mycoplasma hyopneumoniae strain VPP-11, strain J, or P-57223 is grown in 3.5 liters of Friis medium to a density of approximately 10° to centrifugation, washed four times in phosphate buffered saline (PBS), and lysed in 10 mM Tris pH 8.0, 10 mM EDTA by repeated freeze-thaw cycles. Debris is removed by centrifugation at 12,000 g for 10 minutes. The membranes are harvested by centrifugation at 105,000g for 45 minutes, washed once in PBS, and solubilized by 1.0% Triton X-100 in PBS for 18 hours at 4!C. The solubilized material is fractionated by gel filtration using Sepharose S-200 and the active peak pooled. The enzymatic activity is purified by affinity chromatography on DNA-cellulose equilibrated with 25 mM Triis pH 8.0 30 mM sodium chloride, 1 mM EDTA, 1 mM dithiothreitol, 0.3% Triton X-100, 3 mM sodium azide, washed extensively with the same buffer, and eluted with 1.0M sodium chloride in the same buffer. By SDS polyacrylamid-gel electrophoresis the enzyme is a doublet of 37/39 kDa.

### EXAMPLE 2

Mycoplasma hyopneumoniae strain VPP-11, strain J, or P-57223 is grown in 1.0 liter of Friis medium to a density of approximately 10⁹ to 10¹⁰ color changing units per ml. The cells are removed by centrifugation and 231 grams solid ammonium sulphate is added to the supernatant on ice to achieve 40% saturation. The protein precipitate is harvested by centrifugation, resuspended in 17.5 mM potassium phosphate pH 7.0, 3 mM sodium azide and dialyzed extensively against the same buffer. The activity is further purified by ion-exchange chromatography on DEAE-cellulose where the active fraction does not bind. The enzymatic activity is purified by affinity chromatography on DNA-cellu!ose equilibrated with 25 mM Tris pH 8.0, 30 mM sodium chloride, 1 mM EDTA, 1 mM dithiothreitol, 0.3% Triton X-100, 3 mM sodium azide, washed extensively with the same buffer, and eluted with 1.0 M sodium chloride in the same buffer. This is the DNA-cellulose fraction. By SDS polyacrylamide-gel eletrophoresis the enzyme is a doublet of 37/39 kDa.

### EXAMPLE 3

A 6 ml aliquot (total volume: 12 ml) of the DNA-cellulose fraction from Example 2 was combined with an equal volume of Freund's incomplete adjuvant through dropwise addition of the adjuvant while the resulting homogenate was constantly mixed by sonication.

### Vaccinations

Miniature pigs are immunized sub-cutaneously in the hip with 2.0 ml aliquots of the vaccine plus adjuvant at the age of seven days, with a booster injection administered two weeks after the initial injection.

### Challenge

Another group of pigs of the same age as the experimental animals were intratracheally innoculated with P-57223 M. hyopneumaniae at the age of three days are served as "seeder pigs" for the subsequent swine mycoplasmal pneumonia (MPS) challenge. Three weeks after the initial vaccination with experimental animals described above were commingled with the seeder pigs to initiate the transmission of M. hyopneumoniae by contact exposure. The animals remained together until necropsy, six weeks after the initiation of MPS challenge.

In the 6 non-vaccinates, there were two pigs with MPS lesions and four pigs with no lesions. In the 6 vaccinates, there were no pigs with MPS lesions and six pigs with no lesions.

### Example 4 (Preparation of antibody)

M. hyopneumoniae strain P-57223 was grown in 1 liter of Friis medium to a density of approximately 10^{∼} to 10*^{∼} color changing units per ml. The cells were removed by centrifugation and solid ammonium sulphate was added to the growth culture supernatant to achieve 40% saturation at 0!C. The protein precipitate was harvested by centrifugation, resuspended in 17.5 mM potassium phosphate pH 7.0, 3 mM sodiumr azide and dialyzed extensively against the same buffer. The activity was further purified by ion-exchange chromatography on DEAE-cellulose and by affinity chromatography on DNA-cellulose as described previously.

Miniature pigs were immunized with 200 micrograms of DNA cellulose fraction in incomplete Freund's adjuvant. A booster injection was administered 2 weeks after the initial injection and thepigs were bled 2 weeks later. By Western blot analysis of total M. hyopneumoniae proteins, the antisera were shown to react at a 1:50 dilution with at least 6 proteins with apparent molecular weights between 26 and 97 kDa.

Such antisera may be employed in formulating a vaccine as hereinabove described.

### Example 5

### Preparation of DNase

Mycoplasma hyopneumoniae strain P-57223 was grown in 25 liters of Friis medium to ≧10¹⁰ color changing units per ml. Cells were harvested by centrifugation and discarded. The culture supernatant was dialyzed at 4°C against 25 mM Tris, pH 8.0, 1mM MgCl₂, 1mM CaCl₂, 1mM dithiothreitol and passed over Whatman DE 52 DEAE-cellulose in the same buffer. The unbound fraction was collected and adjusted to 50mM Tris, pH8.0, 5mM MgCl₂, 5mM CaCl₂, 5mM dithiothreitol. Solid ammonium sulfate was added to 0°C to achieve 55% saturation. Insoluble material was harvested by centrifugation and discarded. Solid ammonium sulfate was added to the centrifugation supernatant at 0°C to achieve 90% saturation. Insoluble protein was harvested by centrifugation, resuspended in cold 50mM Tris, pH 8.0, 5mM MgCl₂, 5 mM dithiothreitol, 0.1% CHAPS (w/v), dialyzed at 4°C against the same buffer, and passed over Whatman P 11 phospho-cellulose at 4°C. The phospho-cellulose column was eluted with 50mM Tris, pH8.0, 5mM MgCl₂, 5mM CaCl₂, 5mM dithiothreitol, 0.1% CHAPS (w/v), 0.5 M KCl at 4°C. The eluted material was dialyzed against 50mM sodium acetate, pH 4.0, 0.1% CHAPS (w/v) and passed over DNA-Sepharose (160 mg. E. coli DNA per ml of Pharmacia Sepharose 6B resin) at 4°C. The DNA-Sepharose column was eluted at 4°C first with 50mM sodium acetate, pH 4.0, 0.1% CHAPS, 0.2M NaCl, then with 50mM sodium acetate, pH 4.0, 0.1% CHAPS (w/v), 1.0M MgCl₂. The material from the final elution was pooled as the product.

## Claims (Claims for the following Contracting State(s): AT, BE, CH, DE, FR, GB, IT, LU, NL, SE)

1. A composition for protecting an animal against a disease caused by a microorganism which lacks biosynthetic pathways for nucleic acid precursors and which is a mycoplasma, acholeplasma, spiroplasma, ureaplasma or anaeroplasma, comprising:
(a) a member selected from (i) nucleases which produce antibodies having paratopes which recognise an extrinsic nuclease of the microorganism, and (ii) antibodies having paratopes which recognise an extrinsic nuclease of the microorganism; and
(b) a physiologically-acceptable carrier.

2. The composition of claim 1, wherein the nuclease is a DNase.

3. The composition of claim 2, wherein the DNase is a DNA endonuclease.

4. The composition of claim 2 or claim 3, in unit dosage form, each containing at least 5 µg of the DNase.

5. The composition of any preceding claim, wherein the microorganism is a mycoplasma.

6. The composition of claim 5, wherein the microorganism is M. hyopneumoniae.

7. The composition of claim 6, wherein the member is (i), and which additionally comprises one or more proteins which are capable of eliciting an antibody which recognises a M. hyopneumoniae antigen which lacks immunosuppressive activity.

8. The composition of any preceding claim, wherein the animal is a swine.

9. Use of a material for the preparation of a medicament for protecting an animal against a disease caused by a microorganism which lacks biosynthetic pathways for nucleic acid precursors, the material being a member selected from (i) nucleases which produce antibodies having paratopes which recognise an extrinsic nuclease of the microorganism, and (ii) antibodies having paratopes which recognise an extrinsic nuclease of the microorganism.

10. Use according to claim 9, wherein the nuclease is as defined in claim 2 or claim 3.

11. Use according to claim 9 or claim 10, wherein the microorganism is as defined in any of claims 1, 5 and 6.

12. Use according to claim 11, wherein the microorganisms is M. hyopneumoniae, the use being in combination with one or more proteins as defined in claim 7, wherein the disease is caused by M. hyopneumoniae which lacks biosynthetic pathways for nucleic acid precursors.

13. Use according to any of claims 9 to 12, wherein the animal is a swine.

## Claims (Claims for the following Contracting State(s): ES, GR)

1. A method for making a composition for protecting an animal against a disease caused by a microorganism which lacks biosynthetic pathways for nucleic acid precursors and which is a mycoplasma, acholeplasma, spiroplasma, ureaplasma or anaeroplasma, the method comprising formulating:
(a) a member selected from (i) nucleases which produce antibodies having paratopes which recognise an extrinsic nuclease of the microorganism, and (ii) antibodies having paratopes which recognise an extrinsic nuclease of the microorganism; and
(b) a physiologically-acceptable carrier.

2. The method of claim 1, wherein the nuclease is a DNase.

3. The method of claim 2, wherein the DNase is a DNA endonuclease.

4. The method of claim 2 or claim 3, wherein the composition is in unit dosage form, each containing at least 5 µg of the DNase.

5. The method of any preceding claim, wherein the microorganism is a mycoplasma.

6. The method of claim 5, wherein the microorganism is M. hyopneumoniae.

7. The method of claim 6, wherein the member is (i), and wherein formulation additionally comprises one or more proteins which are capable of eliciting an antibody which recognises a M. hyopneumoniae antigen which lacks immunosuppressive activity.

8. The method of any preceding claim, wherein the animal is a swine.

9. Use of a material for the preparation of a medicament for protecting an animal against a disease caused by a microorganism which lacks biosynthetic pathways for nucleic acid precursors, the material being a member selected from (i) nucleases which produce antibodies having paratopes which recognize an extrinsic nuclease of the microorganism, and (ii) antibodies having paratopes which recognise an extrinsic nuclease of the microorganism.

10. Use according to claim 9, wherein the nuclease is as defined in claim 2 or claim 3.

11. Use according to claim 9 or claim 10, wherein the microorganism is as defined in any of claims 1, 5 and 6.

12. Use according to claim 11, wherein the microorganisms is M. hyopneumoniae, the use being in combination with one or more proteins as defined in claim 7, wherein the disease is caused by M. hyopneumoniae which lacks biosynthetic pathways for nucleic acid precursors.

13. Use according to any of claims 9 to 12, wherein the animal is a swine.

## Patentansprüche (Patentansprüche für folgende(n) Vertragsstaat(en): AT, BE, CH, DE, FR, GB, IT, LU, NL, SE)

1. Zubereitung zum Schutz eines Tieres gegen eine durch einen Mikroorganismus, dem es an Biosynthesewegen für Nucleinsäurevorläufer ermangelt und bei dem es sich um ein Mycoplasma, Acholeplasma, Spiroplasma, Ureoplasma oder Anaeroplasma handelt, hervorgerufene Krankheit, umfassend:
(a) eine Komponente, ausgewählt aus (i) Nucleasen, die Antikörper mit eine äußere Nuclease des Mikroorganismus erkennenden Paratopen produzieren, und (ii) Antikörpern mit eine äußere Nuclease des Mikroorganismus erkennenden Paratopen und
(b) einen physiologisch akzeptablen Träger.

2. Zubereitung nach Anspruch 1, wobei die Nuclease aus einer DNase besteht.

3. Zubereitung nach Anspruch 2, wobei die DNase aus einer DNA-Endonuclease besteht.

4. Zubereitung nach Anspruch 2 oder 3 in Einheitsdosisform, jeweils enthaltend mindestens 5 µg der DNase.

5. Zubereitung nach einem der vorhergehenden Ansprüche, wobei der Mikroorganismus aus einem Mycoplasma besteht.

6. Zubereitung nach Anspruch 5, wobei der Mikroorganismus aus M. hypopneumoniae besteht.

7. Zubereitung nach Anspruch 6, mit der Komponente (i) und zusätzlich einem oder mehreren Protein(en) mit der Fähigkeit zum Herauslocken eines Antikörpers, der ein M. hypopneumoniae-Antigen ohne immunsuppressive Aktivität erkennt.

8. Zubereitung nach einem der vorhergehenden Ansprüche, wobei das Tier ein Schwein ist.

9. Verwendung eines Materials zur Herstellung eines Medikaments zum Schutz eines Tieres gegen eine durch einen Mikroorganismus ohne Biosynthesewege für Nucleinsäurevorläufer hervorgerufene Krankheit, wobei das Material aus einer Komponente, ausgewählt aus (i) Nucleasen, die Antikörper mit eine äußere Nuclease des Mikroorganismus erkennenden Paratopen produzieren und (ii) Antikörpern mit eine äußere Nuclease des Mikroorganismus erkennenden Paratopen besteht.

10. Verwendung nach Anspruch 9, wobei die Nuclease entsprechend Anspruch 2 oder 3 definiert ist.

11. Verwendung nach Anspruch 9 oder 10, wobei der Mikroorganismus nach einem der Ansprüche 1, 5 und 6 definiert ist.

12. Verwendung nach Anspruch 11, wobei der Mikroorganismus aus M. hypopneumoniae besteht, die Verwendung in Kombination mit einem oder mehreren Protein(en) gemäß der Definition von Anspruch 7 erfolgt und die Krankheit durch M. hypopneumoniae, dem es an Biosynthesewegen für Nucleinsäurevorläufer ermangelt, hervorgerufen ist.

13. Verwendung nach einem der Ansprüche 9 bis 12, wobei das Tier ein Schwein ist.

## Patentansprüche (Patentansprüche für folgende(n) Vertragsstaat(en): ES, GR)

1. Verfahren zur Herstellung einer Zubereitung zum Schutz eines Tieres gegen eine durch einen Mikroorganismus, dem es an Biosynthesewegen für Nucleinsäurevorläufer ermangelt und der aus einem Mycoplasma, Acholeplasma, Spiroplasma, Ureoplasma oder Anaeroplasma besteht, hervorgerufene Krankheit durch Vereinigen:
(a) einer Komponente, ausgewählt aus (i) Nucleasen, die Antikörper mit eine äußere Nuclease des Mikroorganismus erkennenden Paratopen produzieren, und (ii) Antikörpern mit eine äußere Nuclease des Mikroorganismus erkennenden Paratopen mit
(b) einen physiologisch akzeptablen Träger.

2. Verfahren nach Anspruch 1, wobei die Nuclease aus einer DNase besteht.

3. Verfahren nach Anspruch 2, wobei die DNase aus einer DNA-Endonuclease besteht.

4. Verfahren nach Anspruch 2 oder 3, wobei die Zubereitung in Einheitsdosisform vorliegt und jeweils mindestens 5 µg der DNase enthält.

5. Verfahren nach einem der vorhergenden Ansprüche, wobei der Mikroorganismus aus einem Mycoplasma besteht.

6. Verfahren nach Anspruch 5, wobei der Mikroorganismus aus M. hypopneumoniae besteht.

7. Verfahren nach Anspruch 6, wobei die Komponente (i) und zusätzlich ein oder mehrere Protein(e) mit der Fähigkeit zum Herauslocken eines Antikörpers, der ein M. hypopneumoniae-Antigen ohne immunsuppressive Aktivität erkennt, zugesetzt wird (werden).

8. Verfahren nach einem der vorhergehenden Ansprüche, wobei das Tier ein Schwein ist.

9. Verwendung eines Materials zur Herstellung eines Medikaments zum Schutz eines Tieres gegen eine durch einen Mikroorganismus ohne Biosynthesewege für Nucleinsäurevorläufer hervorgerufene Krankheit, wobei das Material aus einer Komponente, ausgewählt aus (i) Nucleasen, die Antikörper mit eine äußere Nuclease des Mikroorganismus erkennenden Paratopen produzieren und (ii) Antikörpern mit eine äußere Nuclease des Mikroorganismus erkennenden Paratopen besteht.

10. Verwendung nach Anspruch 9, wobei die Nuclease entsprechend Anspruch 2 oder 3 definiert ist.

11. Verwendung nach Anspruch 9 oder 10, wobei der Mikroorganismus nach einem der Ansprüche 1, 5 und 6 definiert ist.

12. Verwendung nach Anspruch 11, wobei der Mikroorganismus aus M. hypopneumoniae besteht, die Verwendung in Kombination mit einem oder mehreren Protein(en) gemäß der Definition Anspruch 7 erfolgt, wobei die Krankheit durch M. hypopneumoniae, dem es an Biosynthesewegen für Nucleinsäurevorläufer ermangelt, hervorgerufen ist.

13. Verwendung nach einem der Ansprüche 9 bis 12, wobei das Tier ein Schwein ist.

## Revendications (Revendications pour l'(les) Etat(s) contractant(s) suivant(s): AT, BE, CH, DE, FR, GB, IT, LU, NL, SE)

1. Composition destinée à protéger un animal contre une maladie provoquée par un microorganisme qui est dépourvu des voies de biosynthèse des précurseurs d'acides nucléiques et qui est un mycoplasme, un acholéplasme, un spiroplasme, un uréaplasme ou un anaéroplasme, comprenant :
(a) un membre du groupe consistant en (i) des nucléases qui provoquent la production d'anticorps ayant des paratopes qui reconnaissent une nucléase extrinsèque du microorganisme ; et (ii) des anticorps ayant des paratopes qui reconnaissent une nucléase extrinsèque du microorganisme ;
(b) un support physiologiquement acceptable.

2. Composition suivant la revendication 1, dans laquelle la nucléase est une DNase.

3. Composition suivant la revendication 2, dans laquelle la DNase est une endonucléase de dégradation de l'ADN.

4. Composition suivant la revendication 2 ou la revendication 3, sous une forme posologique unitaire, chacun contenant au moins 5 µg de la DNase.

5. Composition suivant l'une quelconque des revendications précédentes, dans laquelle le microorganisme est un mycoplasme.

6. Composition suivant la revendication 5, dans laquelle le microorganisme est M. hyopneumoniae.

7. Composition suivant la revendication 6, dans laquelle le membre est choisi dans le groupe (i), et qui comprend en outre une ou plusieurs protéines qui sont capables d'engendrer un anticorps qui reconnaît un antigène de M. hyopneumoniae qui est dépourvu d'activité immunosuppressive.

8. Composition suivant l'une quelconque des revendications précédentes, dans laquelle l'animal est un porc.

9. Utilisation d'une substance pour la préparation d'un médicament destiné à protéger un animal contre une maladie provoquée par un microorganisme qui est dépourvu des voies de biosynthèse des précurseurs d'acides nucléiques, la substance étant un membre du groupe consistant en (i) des nucléases qui produisent des anticorps ayant des paratopes qui reconnaissent une nucléase extrinsèque du microorganisme, et (ii) des anticorps ayant des paratopes qui reconnaissent une nucléase extrinsèque du microorganisme.

10. Utilisation suivant la revendication 9, dans laquelle la nucléase répond à la définition suivant la revendication 2 ou la revendication 3.

11. Utilisation suivant la revendication 9 ou la revendication 10, dans laquelle le microorganisme répond à la définition suivant l'une quelconque des revendications 1, 5 et 6.

12. Utilisation suivant la revendication 11, dans laquelle le microorganisme est M. hyopneumoniae, l'utilisation étant effectuée en association avec une ou plusieurs protéines répondant à la définition suivant la revendication 7, dans laquelle la maladie est provoquée par un M. hyopneumoniae qui est dépourvu des voies de biosynthèse des précurseurs d'acides nucléiques.

13. Utilisation suivant l'une quelconque des revendications 9 à 12, dans laquelle l'animal est un porc.

## Revendications (Revendications pour l'(les) Etat(s) contractant(s) suivant(s): ES, GR)

1. Procédé de préparation d'une composition destinée à protéger un animal contre une maladie provoquée par un microorganisme qui est dépourvu des voies de biosynthèse des précurseurs d'acides nucléiques et qui est un mycoplasme, un acholéplasme, un spiroplasme, un uréaplasme ou un anaéroplasme, procédé comprenant la formulation :
(a) d'un membre du groupe consistant en (i) des nucléases qui provoquent la production d'anticorps ayant des paratopes qui reconnaissent une nucléase extrinsèque du microorganisme ; et (ii) des anticorps ayant des paratopes qui reconnaissent une nucléase extrinsèque du microorganisme ;
(b) un support physiologiquement acceptable.

2. Procédé suivant la revendication 1, dans lequel la nucléase est une DNase.

3. Procédé suivant la revendication 2, dans lequel la DNase est une endonucléase de dégradation de l'ADN.

4. Procédé suivant la revendication 2 ou la revendication 3, dans lequel la composition est sous une forme posologique unitaire, chacune contenant au moins 5 µg de la DNase.

5. Procédé suivant l'une quelconque des revendications précédentes, dans lequel le microorganisme est un mycoplasme.

6. Procédé suivant la revendication 5, dans lequel le microorganisme est M. hyopneumoniae.

7. Procédé suivant la revendication 6, dans lequel le membre est choisi dans le groupe (i), et dans lequel la formulation comprend en outre une ou plusieurs protéines qui sont capables d'engendrer un anticorps qui reconnaît un antigène de M. hyopneumoniae qui est dépourvu d'activité immunosuppressive.

8. Procédé suivant l'une quelconque des revendications précédentes, dans lequel l'animal est un porc.

9. Utilisation d'une substance pour la préparation d'un médicament destiné à protéger un animal contre une maladie provoquée par un microorganisme qui est dépourvu des voies de biosynthèse des précurseurs d'acides nucléiques, la substance étant un membre du groupe consistant en (i) des nucléases qui produisent des anticorps ayant des paratopes qui reconnaissent une nucléase extrinsèque du microorganisme, et (ii) des anticorps ayant des paratopes qui reconnaissent une nucléase extrinsèque du microorganisme.

10. Utilisation suivant la revendication 9, dans laquelle la nucléase répond à la définition suivant la revendication 2 ou la revendication 3.

11. Utilisation suivant la revendication 9 ou la revendication 10, dans laquelle le microorganisme répond à la définition suivant l'une quelconque des revendications 1, 5 et 6.

12. Utilisation suivant la revendication 11, dans laquelle le microorganisme est M. hyopneumoniae, l'utilisation étant effectuée en association avec une ou plusieurs protéines répondant à la définition suivant la revendication 7, dans laquelle la maladie est provoquée par un M. hyopneumoniae qui est dépourvu des voies de biosynthèse des précurseurs d'acides nucléiques.

13. Utilisation suivant l'une quelconque des revendications 9 à 12, dans laquelle l'animal est un porc.
